# EUROPEAN PATENT APPLICATION

(11) **EP 4 817 566 A1**
(43) Date of publication of application: **07.10.2026**
(21) Application number: 25382346.2
(22) Date of filing: 04.04.2025
(51) Int. Cl.: A61F 2/72

(54) **IMPLANT DEVICE**

(71) Applicant: Fundación de investigación biomédica, Hospital Universitario de Getafe, 28905 Getafe Madrid (ES); Universidad Pontificia Comillas, 28015 Madrid (ES)
(72) Inventor: MALDONADO MORILLO, Andrés Alfonso, 28905 Madrid (ES); GIANNETTI, Romano, 28015 Madrid (ES); MUÑOZ FRÍAS, José Daniel, 28015 Madrid (ES); SANZ BARBERO, Elisa, 28905 Madrid (ES); ALONSO RIVAS, Eduardo, 28015 Madrid (ES); CRISTÓBAL VELASCO, Lara, 28905 Madrid (ES)
(74) Representative: Hoffmann Eitle

(57) **Abstract**

The present invention refers to the technical field of the implant devices, in particular for the application of VDMTs in bionic prosthetics and orthoses including exoskeletons for facilitating the capture of electrical signals of an injured nerve stump to restore motor function after serious injuries.

## Description

### TECHNICAL FIELD

The present invention refers to the technical field of the implant devices, in particular for the application of Vascularized Denervated Muscle Targets (VDMTs) in bionic prosthetics and orthoses including exoskeletons for facilitating the capture of electrical signals of an injured nerve stump to restore motor function after serious injuries.

### BACKGROUND ART

Injuries affecting the entire brachial plexus are considered the most severe, as they lead to significant functional limitations and disabilities in patients, with very limited treatment options available. Loss of hand function is one of the main issues associated with this type of injury. Reconstructive approaches face limitations due to the shortage of suitable donor nerves and the considerable distance between the functional structures needing reconstruction and their possible connection points.

Currently, several techniques aim to restore hand function, though all yield limited functional outcomes. Among them, the contralateral C7 technique connects the contralateral healthy C7 root to the recipient nerve via a retroesophageal route, speeding recovery compared to the subcutaneous path. This connection between the contralateral healthy C7 root and the damage nerve would provide a non-intuitive control of the damage muscle. Patients have typically to active the contralateral arm to active the muscle of the injured side. The double gracilis procedure, popularized by Doi, is performed in three stages and includes muscle and nerve transfers to restore shoulder stability, elbow flexion, and hand sensory function. The Mayo Clinic developed a simplified version of this procedure, involving a single gracilis muscle transfer for elbow flexion and hand prehension. Recently, Oskar Aszmann introduced bionic reconstruction, integrating surgical techniques with myoelectric prosthetics. This two-stage approach utilizes electromyographic signals in the forearm for prosthetic control and, if needed, incorporates nerve and muscle transfers, followed by hand amputation and the fitting of a mechatronic prosthesis using EMGs sensor above the skin.

Regenerative Peripheral Nerve Interface (RPNI) technique, which uses non-vascularized muscle grafts represent a technique aiming to revolutionize the field of bionic prostheses by establishing more efficient communication between the nervous system and external devices while preventing the formation of neuromas. This technique involves surgically grafting a small muscle segment from the patient, usually from an area with low functionality, onto a region near a damaged nerve or a residual limb. The regenerative nature of peripheral nerves is expected to enable the growth, reinnervation, and revascularization of the muscle graft in approximately two to four months, resulting in the formation of a biological amplifier system that translate the nervous signals into myoelectrical ones, that aims to facilitate the transmission of signals between the patient's nervous system and prosthetic devices.

The use of animal models is essential for the advancement of biomedical research. RPNI technique has been thoroughly evaluated in various animal models, primarily in the murine model, yielding highly promising histological and neurophysiological results. At the histological level, these studies have demonstrated muscular neoangiogenesis and axonal regeneration, with minimal fibrosis presence. In addition, considerable magnitude muscle action potentials (motor unit potentials or MUPs) have been recorded. These recordings have facilitated effective transmission of electrical signals between the RPNI and the prosthesis. The RPNI technique has been evaluated in rats and macaques. The significant variability among species prevents any single type of animal from being an ideal model for research related to humans, as no species can fully replicate all human characteristics. Therefore, the use of multiple animal models becomes essential to minimize the disparities between each model and humans, enabling the detection of anomalies directly related to the selected animal model. Large animal models, such as primates, are valuable in medical research due to their similarity to humans in various genetic and physiological aspects. However, their high maintenance and development costs, along with ethical considerations, are significant disadvantages to consider when choosing between large and small animal models for medical studies.

Although the promising results in early studies with animals, latest studies, as shown in example 2 revealed that achieving adequate neovascularization of the muscle graft is not so straightforward. Vascularization is fundamental to ensuring muscle tissue survival and, consequently, the effectiveness of the technique.

There is therefore a need for an alternative technique to RPNI that can provide consistent results in terms of efficient communication between the nervous system and external devices while preventing the formation of neuromas, without the neovascularization issues shown by RPNI.

### BRIEF DECRIPTION OF THE FIGURES

**Figure 1****.** Anatomical references.
**Figure 2****.** Skin incision.
**Figure 3****.** Omohyoid muscle.
**Figure 4****.** Transverse cervical artery.
**Figure 5****.** Phrenic nerve.
**Figure 6****.** C5 and C6 roots and upper trunk.
**Figure 7****.** Middle trunk.
**Figure 8****.** C8 and T1 roots and lower trunk.
**Figure 9****.** Longitudinal incision.
**Figure 10****.** Pectoralis major flap.
**Figure 11****.** Vascular pedicle.
**Figure 12****.** Pectoralis major pedicle flap.
**Figure 13****.** Device in the pectoralis muscle flap.
**Figure 14****.** Device in the pectoralis muscle flap.
**Figure 15****.** Diagram of a method for isolating the data of the electrical signals generated by a reinnervated VDMT of a subject according to one or more embodiments of the invention.
**Figure 16****.** Comparison of histological findings in rabbits subjected to RPNI, with subjects (rabbits #1-#4) arranged on the X-axis and the evaluated histological parameters on the Y-axis.
**Figure 17****.** Comparison of histological findings in rabbits subjected to VDMT, with subjects (rabbits #5-#8) arranged on the X-axis and the evaluated histological parameters on the Y-axis.

It should be noted that the figures set forth herein are intended to exemplify the general characteristics of invention, among those of the present disclosure, for the purpose of the description of certain embodiments. These figures may not precisely reflect the characteristics of any given embodiment, and are not necessarily intended to fully define or limit specific embodiments within the scope of this disclosure.

### DETAILED DESCRIPTION OF THE INVENTION.

Elective amputation with reconstruction using myoelectric prosthetics is considered an effective treatment for selected patients with traumatic injuries including traumatic brachial plexus injuries where surgical reconstruction cannot further improve the function. This intervention has demonstrated not only a reduction in mechanical pain but also an increase in employment rates and a high level of satisfaction among postoperative patients. Furthermore, in amputees with adequate non-intuitive electromyographic signals, myoelectric prosthetics have facilitated the opening and closing of the hand, resulting in high rates of prosthesis use. In this regard, bionic reconstruction demonstrates great potential for improving hand function in the coming years following panplexus injury. However, this recent concept has some limitations:
1. The signals are not intuitive, as they originate from muscles unrelated to the natural function of opening or closing the hand. As a result, the patient must undergo a learning process to adapt and control the prosthesis effectively.
2. The prosthesis lacks a direct connection to the brain, which means there is no proprioception or sensory feedback. Consequently, the patient must rely on constant visual monitoring to operate the prosthesis accurately.

We herein propose capturing signals directly from the injured nerve roots (C8 and T1, which are responsible for hand movement) using implantable devices, with the aid of vascularized muscle tissue acting as a biological amplifier. These nerve roots are often avulsed from the spinal cord, meaning that they are anatomically disconnected and theoretically non-functional. However, our recent clinical observations suggest that, even in such cases, a small number of axons may remain in continuity with the spinal cord. In patients with complete root avulsions confirmed by MRI, we have observed a delayed recovery of hand proprioception, understood as the ability to perceive whether the fingers are open or closed. This clinical finding supports the hypothesis that, despite radiological evidence of complete avulsion, a subset of axons may remain in continuity with the spinal cord. These residual axons could potentially be used to reinnervate a vascularized denervated muscle, which would then amplify the minimal electrical signal. This amplified signal could be captured by an implantable device and utilized to control a prosthetic or orthotic system.

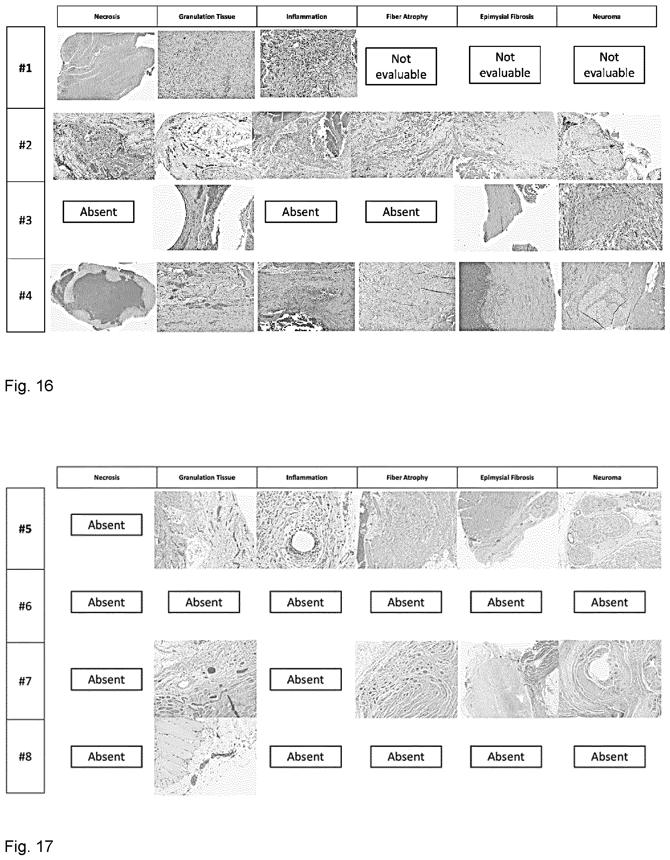

Vascularized Denervated Muscle Targets (VDMTs) could be a valuable option in restoring function in cases of complete brachial plexus injuries, particularly in the area of myoelectric orthoses. Unlike the traditional Regenerative Peripheral Nerve Interface (RPNI) technique, which uses non-vascularized muscle grafts, see Figure 16, VDMTs involve the creation of a denervated muscle flap that remains vascularized, see Figure 17. This flap is strategically placed around the affected nerve stump, effectively overcoming the viability and size limitations associated with conventional grafts. The nerve stump can be divided in different fascicles, or not (depending the number of macroscopically viable fascicle). Each fascicle can be attached to different parts of the VDMTs.

VDMTs facilitates the connection between these devices and the nerve, enabling a more effective functional recovery and potentially improving the quality of life for patients with complete brachial plexus injuries. By using the same nerve stump that used to control the muscles, a more natural control of the exoskeleton could be obtained. The integration of technology with surgical techniques represents an innovative approach that has the potential to transform the treatment of these severe injuries. The application of VDMTs in bionic prosthetics / exoskeleton would facilitate the capture of electrical signals at the brachial plexus roots, even the damaged ones. The worse scenario for a root is an avulsion (the root is not connected to the spinal cord). However, as mentioned above, it has been proven that even in cases of root avulsion, some axons are still alive and connected to the spinal cord, and are able to connect to the VDMT. The VDMT would therefore function as "amplifier" of any electrical signal remaining, even if their amplitude is reduced when compared to a healthy root. The implantable device can potentially pick up the signal from the VDMT and sent to an exoskeleton or myoelectric prothesis. This capability to obtain intuitive muscular electrical signals more effectively not only enhances prosthetic control but also optimizes patients' quality of life by allowing a more intuitive and efficient use of these devices.

One of the main challenges in constructing VDMT lies in identifying the most suitable muscle for its implementation, given that this choice must consider the proximity and accessibility of the brachial plexus roots and trunks, as well as the functional limitations resulting from the partial or total loss of function in the donor site. Selecting the ideal muscle is critical, as any functional alteration in the donor muscle can significantly impact the patient's overall rehabilitation and the effectiveness of the VDMT in capturing myoelectric signals.

For these reasons, we conducted a cadaveric study (see example 1) aimed at evaluating which specific muscles could be used as potential options for performing a VDMT from the C8 and T1 nerve roots. Both roots control the hand, they are typically avulse in brachial plexus injuries, and they have no options to recover using standard reconstructive surgery techniques because of the long distance between the healthy axons and the muscles controlling the hand. It is made plausible throughout the present specification that a VDMT placed on these roots could be useful in connecting a myoelectric orthosis or prosthesis, offering an innovative alternative to enhance functionality in cases of complete brachial plexus injury.

This cadaveric study carried out for the present invention (example 1), aimed to identify the most suitable muscle for constructing a VDMT in patients with brachial plexus injuries, focusing on the reconstruction of the C8-T1 root using the VDMT technique with a vascularized pectoralis major muscle flap, which offers significant advantages over nerve transfer and other muscle flaps, particularly in terms of vascular supply and protection of the injured nerve. Muscles around the brachial plexus are potentially damaged due to the energy trauma this zone had suffered. VDMT relies on vascularized and denervated muscle, so the healthy muscles of the contralateral extremity are not suitable. The pectoral muscle is innervated by the damaged brachial plexus. In a patient with a complete plexus injury, this muscle is typically not working due to the loss of innervation. During the dissection, the anterior and middle scalene muscles were identified as the closest to the inferior trunk, with average distances from the C8 roots to the anterior scalene measuring 1.4 cm and to the middle scalene 2.25 cm. However, concerns were raised about the functionality of these muscles following a brachial plexus injury and the impact of dissection on their integrity.

Therefore, it was found to be preferable to select a muscle that is distant from the injury site. In this context, the pectoral muscle flap was proposed as a viable option for constructing the VDMT, emphasizing its reliability and an average length of 9 cm, which is appropriate for this application.

This invention thus highlights, for the first time, the potential of VDMTs as an innovative therapeutic strategy to improve function in patients with complete brachial plexus injuries, especially in relation to bionic prostheses. Cadaveric dissection identified the pectoral muscle flap as a reliable option for creating VDMTs, providing an effective alternative for connecting myoelectric devices to the brachial plexus. The integration of surgical techniques with technological advancements will lead to significant improvements in functional recovery and quality of life for affected patients.

The present invention provides methods and systems for receiving, with processing circuitry of an implant device, electrical signals from Vascularized Denervated Muscle Targets (VDMTs) attached to the nerves of a subject through electrodes, preferably bipolar electrode pairs, each having a positive and a negative electrode, in electrical communication with the VDMTs.

As used herein, the term "Vascularized Denervated Muscle Targets" (VDMTs) refers to any anatomical construct, graft, or muscle segment that retains a viable vascular supply and have no innervation. The muscle tissue is (i) supplied by its native blood vessels (e.g., through a pedicled flap), or (ii) reestablished via microsurgical techniques (e.g., a free flap anastomosed to recipient vessels). The critical requirement is that the tissue remains perfused and capable of sustaining normal metabolic activities by way of an intact or reconnected vascular network. The muscle fibers and associated tissues have substantially reduced or eliminated neural innervation through surgical transection, intentional disruption of nerve supply, or other means, such that the muscle segment does not respond to or transmit native neuromuscular signals. This denervation may be complete or near-complete, depending on the design criteria, while still preserving the structural and vascular integrity of the muscle. The essential feature distinguishing VDMTs from ordinary muscle flaps is the intentional, controlled denervation combined with the preservation or restoration of robust vascularization, thereby creating a biologically active but neurologically "quiet" substrate that can be used to integrate with prosthetic devices. The electrical signals from the VDMTs have a voltage amplitude of greater than or equal to about 50 microvolts. The processing circuitry processes and wirelessly transmits the electrical signals to a prosthetic controller that controls a prosthetic device.

In particular, a first aspect of the invention refers to a system comprising an implant device comprising a processing circuitry and a communication circuitry, configured to receive electrical signals from one or more VDMTs of a subject attached to one or more nerves of the subject through one or more first electrodes, wherein the one or more first electrodes are in electrical communication with the VDMTs, wherein the VDMTs pertain to a donor muscle of the subject. Preferably, the subject has injuries affecting the brachial plexus, preferably the entire brachial plexus.

Preferably, the nerve/s are selected from the list consisting of:
- Median Nerve (C5-T1 roots) arising from both the lateral and medial cords of the brachial plexus, the median nerve innervates most forearm flexor muscles (except flexor carpi ulnaris and the medial part of flexor digitorum profundus) and several hand muscles (including the thenar group and lateral lumbricals). It also carries sensory information from the palmar side of the thumb, index, middle, and half of the ring finger. Due to its key role in fine motor control and sensation, this nerve is often targeted in bionic devices aiming to restore hand function.
- Ulnar Nerve (C8-T1 roots). Originating from the medial cord of the brachial plexus, the ulnar nerve innervates most intrinsic hand muscles (interossei, the medial two lumbricals, adductor pollicis) and the flexor carpi ulnaris and medial portion of flexor digitorum profundus in the forearm. It provides sensory input from the little finger and the adjacent half of the ring finger (palmar and dorsal aspects). Because it governs fine finger movements and grip strength, the ulnar nerve is a crucial target for bionic limb control and sensory feedback.
- Radial Nerve (C5-T1 roots). Derived from the posterior cord of the brachial plexus, the radial nerve powers the extensor muscles of the elbow, wrist, and fingers, enabling extension and release movements. It also supplies sensation to much of the posterior arm, forearm, and part of the dorsum of the hand. In bionic devices, capturing or stimulating radial nerve signals restores important extension actions, contributing to a more natural range of motion.
- Musculocutaneous Nerve (C5-C7 roots). Branching from the lateral cord of the brachial plexus, this nerve primarily innervates the flexor muscles in the anterior compartment of the arm (biceps brachii, brachialis, coracobrachialis) and continues as the lateral cutaneous nerve of the forearm for sensory innervation. Though it is less commonly used for hand-specific bionic control, it can be valuable for elbow flexion and certain myoelectric signal pickups in above-elbow prosthetic implementations.
- Axillary Nerve (C5-C6 roots). Emanating from the posterior cord, the axillary nerve innervates the deltoid and teres minor muscles and provides sensation to the "regimental badge" area over the lateral shoulder. While it is primarily involved in shoulder abduction and external rotation, it may be relevant for higher-level prosthetics that incorporate shoulder movement or require myoelectric signals from the shoulder region.

These nerves, alone or in combination, can be leveraged for the implant device of the present invention that pick up motor commands (efferent signals) and translate them into prosthetic movement, compensating for loss of function due to brachial plexus injuries.

As used herein "processing circuitry" shall be understood as any subsystem of electronic components comprising at least a processor, and preferably a memory, configured to receive, process, and transmit data and control signals within the system. It is noted that the processor may comprise one or more processing units, such as a microprocessor, GPU, CPU, multi-core processor or the like. Similarly, the memory may comprise one or more volatile or non-volatile memory devices, such as DRAM, SRAM, flash memory, read-only memory, ferroelectric RAM, hard disk drives, floppy disks, magnetic tapes, optical disks or the like.

As used herein "implant device" shall be understood as any medical device designed to be inserted, embedded, and/or affixed within the body, typically for therapeutic, diagnostic or monitoring purposes. In the present invention, the implant device is preferably a device which comprises a printed circuit board configured with a system-on-chip processor and communication circuitry, an analog front end for signal conditioning, an energy source, and a charging and status monitoring system. The processor is configured to execute instructions and support wired and/or wireless communications for data transmission and remote configuration, including over-the-air firmware updates. The analog front end is configured to amplify and filter signals, and the energy source is configured to supply power to the implant device. The charging and monitoring system is configured to replenish the energy source and track the operational status of the implant device. Possible implementations include using a low-power microcontroller with built-in Bluetooth Low Energy (BLE) for wireless communication, a dedicated analog front-end integrated within the SoC or as a separate module, and a lithium-ion or lithium-polymer battery as the energy source. The charging system may be designed as an inductive charger for a completely sealed implant environment or as a physical connector that enables wired charging during clinical maintenance.

As used herein, the term "subject" refers to any human or non-human animal that may benefit from, or is otherwise suitable for, the devices and methods described in this application. While in many embodiments the subject is a human patient in need of medical intervention, veterinary applications are also contemplated. Unless otherwise specified, the term "subject" is inclusive of all mammalian species, including but not limited to humans, companion animals (e.g., dogs, cats), farm animals, laboratory animals, and non-human primates.

As used herein "prosthetic device" shall be understood as any device configured to restore a function lost by the user, including prostheses, orthoses, exoskeletons, or any other device designed to replace or augment impaired functionality.

As used herein "prosthetic controller" shall be understood as any controller configured to control and regulate the operation of a prosthesis by processing inputs from the one or more electrodes and generate control signals to actuate the prosthetic components. The **controller** may be implemented in software (a computer program), hardware (a physical device), or any combination in order to execute the sequences of operation disclosed herein.

As used herein, "communication circuitry" shall be understood as any subsystem of electronic components configured to transmit or receive signals between the system and external devices such as a prosthetic device. The communication circuitry may use wired or wireless means to transmit or receive the signals. In the present invention, the communication circuitry is preferably configured to transmit signals from the processing circuitry to a prosthetic controller of a prosthetic device or from a prosthetic controller within the system to a prosthetic device.

The present invention thus includes a system comprising an implant device comprising a processing circuitry and a communication circuitry and optionally a prosthetic controller and a prosthetic device. The processing circuitry is configured to receive electrical signals from one or a plurality of VDMTs pertaining to a donor muscle of a subject, preferably surgically, attached or wrapped around one or more nerves of the subject, wherein the electrical signals are received through one or a plurality of first electrodes, preferably bipolar electrode pairs, each having a positive electrode and a negative electrode, that are implanted inside of the one or a plurality of VDMTs and/or in electrical communication with the one or a plurality of VDMTs. The processing circuitry is further configured to process the received electrical signals, generate processed signal data, and to, preferably wirelessly, transmit the processed signal data through the communication circuitry. The nerves of the subject thus reinnervate the one or a plurality of VDMTs. The electrical signals from the one or a plurality of VDMTs have a voltage amplitude of greater than or equal to about 50 microvolts. The prosthetic controller is configured to control the prosthetic device based on the processes signal data, preferably wirelessly, transmitted from the communication circuitry of the implant device. That is, in other embodiments, the communication circuitry is configured to transmit the processed electrical signals to a prosthetic device. In other embodiments, the communication circuitry is configured to transmit the processed signal data to a prosthetic controller of the prosthetic device. In other embodiments, the processing circuitry is configured to generate commands to control the prosthetic device based on the processed electrical signals.

In other embodiments, the implant device is further configured to receive electrical signals from muscle fibres of the donor muscle through one or more second electrodes, preferably bipolar electrode pairs, each having a positive electrode and a negative electrode, which are implanted inside of the donor muscle.

In the first aspect of the invention, the processing circuitry is configured to process the received electrical signals to isolate the data of the electrical signals generated by the reinnervated VDMTs from at least an interference generated by the donor muscle.

In other embodiments, the processing circuitry is configured to process the received electrical signals to isolate the data of the electrical signals generated by the reinnervated VDMTs from electrical signals generated by the donor muscle by:
a) analysing the received signals in term of statistical parameters in the time and frequency domain; and
b) applying deterministic and/or deep-learning based methods to isolate the data of the electrical signals generated by the reinnervated VDMTs from at least an interference generated by the donor muscle.

In various embodiments of the present invention, the implant device includes processing circuitry configured to receive and analyse electrical signals from a reinnervated VDMTs in a subject's residual limb. Importantly, while this muscle has been reinnervated by a new nerve (as part of a surgical or therapeutic procedure), residual innervation or muscle activity from its original nerve supply may still be present and can be picked up by the electrodes in the VDMT. Consequently, the processing circuitry is designed to distinguish or "isolate" the relevant myoelectric signals contributed by the newly reinnervated nerve from those myoelectric signals that do not pertain to the nerve used to reinnervate the muscle.

In these embodiments, electrodes are placed on or in close proximity to the reinnervated muscle so as to detect electrical activity originating from both the newly reinnervated region and any remaining electrical activity within the same muscle. Because signals from both sources are co-located, the raw data stream will contain overlapping electrical activity.

In a first stage, the processing circuitry is configured to process the received electrical signals by analysing them in term of statistical parameters in the time and frequency domain. As part of this stage, the processing circuitry may comprise one or more of the following:
- an amplification means; and/or
- an analog-to-digital converter (ADC).

In this first stage, the processing circuitry may be configured to perform one or more of the following:
- amplify the signals received by the electrodes;
- filter the signals received by the electrodes, either before or after the amplification stage, preferably comprising at least high-pass filtering (for contact potential removal) and antialiasing (for ADC correctness);
- digitize the amplified signals by the ADC at a sampling frequency suitable for capturing the frequency range of muscle activity (e.g. any range included in the 1 Hz- 10 kHz, optimized by the specific muscle/implant pickup); and/or
- apply time- and frequency-domain functions to both signals to parametrise the signals received from the one or more VDMTs and the donor muscle. The system of the invention might calculate metrics such as root mean square (RMS) amplitude, mean absolute value (MAV), variance, pulse repetition frequency, and waveform length. These features help delineate differences in activation patterns between the newly reinnervated regions versus any residual electrical activity. Using methods like Fast Fourier Transform (FFT) or wavelet decomposition, the system can later identify spectral signatures unique to the signals driven by the new nerve, distinguishing them from those generated by the residual electrical activity.

In another embodiment, wherein the implant device is further configured to receive electrical signals from muscle fibres of the donor muscle through one or more second electrodes, the processing circuitry is configured to process the received electrical signals by analysing both signals as received by the first and second electrodes. Advantageously, the signals received by the second electrodes can provide useful data regarding the electrical signals generated by the donor muscle.

Once digitized, in a second stage, the processing circuitry is configured to process the composite signal received from the one or more VDMTs by the first electrode -encompassing both reinnervated and residual muscle activity-to isolate the data of the electrical signals generated by the reinnervated VDMTs through deterministic and/or deep-learning based methods. This allows to identify the user's intended commands - the electrical signals generated by the reinnervated VDMTs - versus extraneous or unwanted interference - generated by the donor muscle.

Because the same muscle may exhibit electrical activity from distinct sources, the processing circuitry may employ advanced isolation techniques in the second stage. The goal is to identify and preserve the signal components specific to the newly reinnervated nerve pathway while filtering out or de-emphasizing signals from the muscle's original and remaining electrical activity.

Traditional signal processing approaches-such as adaptive filtering, principal component analysis (PCA), or independent component analysis (ICA)-may be employed to mathematically separate multiple sources of muscle activation within the same EMG signal. When the implant device is further configured to receive electrical signals from muscle fibres of the donor muscle through one or more second electrodes, the traditional signal processing may comprise, for example, subtracting the donor muscle signal from the VDMTs signal.

More advanced embodiments utilize deep neural networks (DNNs), convolutional neural networks (CNNs), or recurrent neural networks (RNNs) to classify and isolate signals. The algorithms might be trained on labelled data where it is known that the subject has selectively activated the reinnervated nerve pathway (e.g., performing specific muscle contractions post-reinnervation). For example, the subject may perform specific activation patterns to help the system learn and refine signal isolation, ensuring consistent performance across varied tasks. The labelled clean donor and noisy target signals, either in the frequency domain or in the time domain, can be used to train a separation neural network. This network will learn to predict the donor signal from noisy inputs, similar to the process used in speech separation to tell voices apart. The residual signal will be regarded as the clean target signal.

Over time, the algorithm learns characteristic features of these signals, allowing it to filter out signals that stem from residual electrical activity of the same muscle.

In some embodiments, the algorithm continuously updates its parameters-accommodating physiological changes, electrode drift, or variations in the residual muscle's activity-to maintain robust performance in isolating the reinnervated signal source.

The implant device of the invention may incorporate shielded cables, differential amplifiers, and prefilters to minimize external electromagnetic interference (EMI) and to prepare the signal for the digital conversion (anti-aliasing). Digital signal filters (e.g., bandpass or notch filters) can remove frequency components that do not correspond to muscle-derived signals (e.g., 50/60 Hz power line noise). By placing multiple electrodes across the reinnervated VDMTs, the device can leverage spatial discrimination to detect slight variations in signal origin within the same muscle belly.

A data fusion layer may combine information from multiple channels to enhance the reliability of isolating the newly reinnervated nerve signals. Once isolated, the signals from the reinnervated nerve can be used to drive the prosthetic device.

Targeted Muscle Reinnervation (TMR) is a surgical procedure in which residual peripheral nerves from an amputated limb are redirected and connected to nearby motor nerve, to reinnervate a muscle which typically has no more function. These muscles then act as biological amplifiers, producing strong electrical signals in response to nerve activity. While the disclosed technique is particularly beneficial in upper-limb TMR scenarios, it also extends to lower-limb prosthetics, exoskeleton systems, and other bionic devices where a muscle may receive partial reinnervation yet retain some original innervation.

By isolating signals specifically tied to the newly reinnervated nerve pathways, the system yields improved command fidelity and reduces misinterpretations caused by unwanted signals from the same muscle's residual electrical activity.

Advanced filtering and machine learning enable dynamic adaptation to changes in muscle physiology, electrode placement, or user habits over time, maintaining stable performance.

The disclosed implant device can interface with a wide range of prosthetic limbs and exoskeletons, or any other biomedical device reliant on finely parsed myoelectric signals from reinnervated muscles. Precise signal isolation ensures that user intent is translated accurately into prosthetic motion, reducing mental load and improving overall functional outcomes.

Advantageously, through the embodiments described above, the present invention provides robust configurations for extracting user-intended signals from reinnervated muscle tissue that may still be partially residual. By employing statistical analyses and deterministic and/or deep-learning-based algorithms, the system effectively isolates the newly reinnervated signals from non-target activity within the same muscle, thereby enabling higher precision in controlling bionic prosthetics or other implant devices. These teachings are illustrative and not limiting; modifications and variations consistent with the claims are intended to fall within the scope of the invention.

It is also noted that the embodiments of the first aspect of the invention provide a system that can rely on a direct connection between the muscle and the one or more first electrodes (e.g. bipolar needles). Advantageously, in contrast with systems relying on superficial electrodes which read EMG signals through the skin, the embodiments of the first aspect of the invention enable the reception of smaller signals from the one or more VDMTs, including signals from severed damage nerves with low number of viable axons producing a low electrical muscle signal.

In another embodiment, wherein the implant device is further configured to receive electrical signals from muscle fibres of the donor muscle through one or more second electrodes, the processing circuitry is configured to further process the composite signal received from the one or more VDMTs by the first electrode through deterministic and/or deep-learning based methods considering the signal received from the donor muscle. Advantageously, having the signal received from the donor muscle as an input into the algorithm, further facilitates the isolation of the newly reinnervated signals from non-target activity within the same muscle.

In other embodiments, the one or more first electrodes receive signals from one or more VDMTs which have been wrapped around or surgically attached to the nerve to interact with said nerve. Advantageously, our technique of wrapping the nerve with vascularized muscle let to capture any axons that grows into any direction.

In other embodiments, the one or more electrodes are mono-polar or bipolar needle electrodes, or bipolar or monopolar hook wire electrodes, or sub-miniature needle electrodes. Advantageously, hook wire electrodes easily attack to the vascularized denervated muscle target (VDMT). For example, the one or more electrodes are hook wire electrodes with a needle diameter of 0.6 mm.

In other embodiments, the donor muscle is the peroneal, the sternocleidomastoid, the omohyoid, the trapezius or other muscles located in proximity to the brachial plexus. Additionally, free muscle flaps such as the rectus femoris or the gracilis may be used as vascularized denervated targets, depending on the specific anatomical and surgical requirements.

In other embodiments, the implant device includes protection circuitry to protect the implant device from surge voltages during an electrostatic discharge event during implantation of the implant device within a patient.

In other embodiments, the protection circuitry includes at least one resistor and at least one diode.

In other embodiments, the one or a plurality of electrodes, preferably bipolar electrode pairs, include a first set of electrodes and a second set of electrodes, wherein the first and second set of electrodes are each connected to the implant device preferably with first and second multi-contact connectors, respectively, each having a pair of contacts associated with each electrodes and wherein the implant device includes a header with first and second ports configured to receive the first and second multi-contact connectors, respectively.

In other embodiments, the first and second ports each include a Bal Seal connector configured to seal the implant device once the first and second multicontact connectors are inserted into the first and second ports.

In other embodiments, the processing circuitry is configured to generate commands to control the prosthetic device based on the received electrical signals and to generate an estimated command to control the prosthetic device during a time period when the at least one stimulation signal is generated and transmitted to the at least one of the electrodes.

A second aspect of the invention relates to a method for isolating the data of the electrical signals generated by a reinnervated VDMT of a subject, wherein the reinnervated VDMT pertain to a vascularised muscle flap of a donor muscle of the subject, and wherein the muscle fibres have been reinnervated with nerve axons from the subject.

In various embodiments of the present invention, the method includes configuring the processing circuitry to receive and analyse electrical signals from a reinnervated VDMTs in a subject's residual limb. Consequently, the method is aimed at distinguishing or "isolating" the relevant myoelectric signals contributed by the newly reinnervated nerve from those myoelectric signals that do not pertain to the nerve used to reinnervate the muscle.

In these embodiments, electrodes are placed on or in close proximity to the reinnervated muscle so as to detect electrical activity originating from both the newly reinnervated region and any remaining electrical activity within the same muscle.

Because signals from both sources are co-located, the raw data stream will contain overlapping electrical activity.

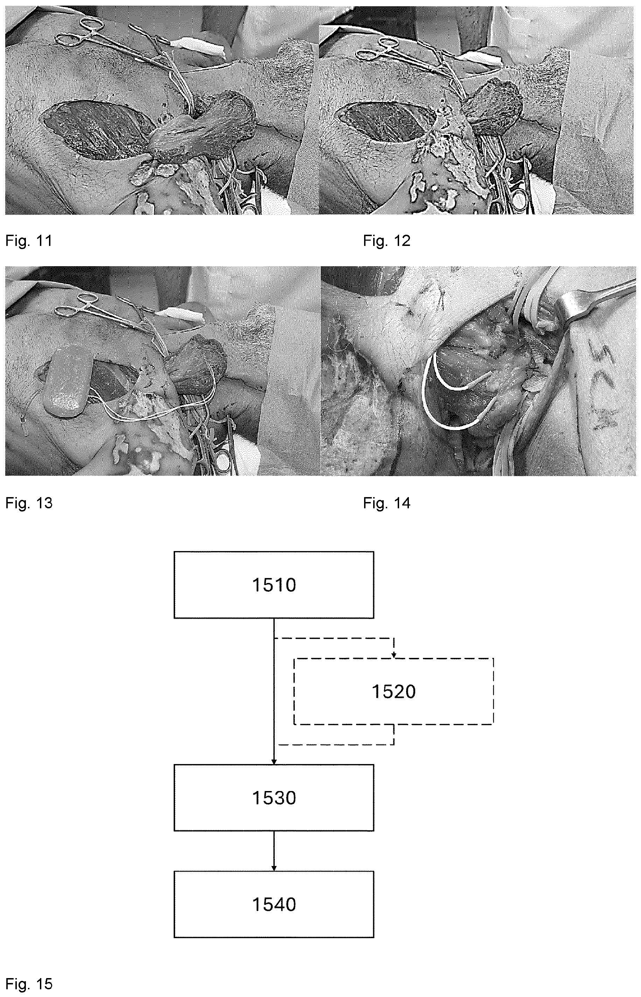

The method comprises the following steps as shown by Figure 15:
In a first step 1510, it involves receiving electrical signals associated to: the reinnervated muscle fibres.

In some embodiments, the first step 1510 involves receiving synchronised electrical signals associated to: the reinnervated muscle fibres and the muscle fibres of the donor muscle. The signals may be synchronised in different ways, for example by ensuring they comprise a timestamp associated with the data, or by an appropriate synchronization protocol as the skilled person may envisage.

In some embodiments the data is received by a processing unit from a memory unit or from an external device through different communication means. For example, the data may be received from a communication circuitry of any recording means, such as an implant according to the first aspect of the invention.

In some embodiments, the signals are received with an implant device having processing circuitry and communication circuitry, electrical signals from one or a plurality of VDMTs attached to nerves of a subject, wherein the electrical signals are received through one or a plurality of electrodes, preferably bipolar electrode pairs, each having a positive electrode and a negative electrode, that are implanted inside of the one or a plurality of VDMTs and/or in electrical communication with the one or a plurality of VDMTs, the one or a plurality of VDMTs being doner muscles from the subject, vascularized, and deinnervated prior to being attached to the nerves of the subject. Therefore, it is submitted that the electrical signals associated to the reinnervated muscle fibres and the muscle fibres of the donor muscle may be received from an implant according to the first aspect of the invention.

In a second step 1520, which may be present in some embodiments, the method further includes, preferably wirelessly, transmitting, with the communication circuitry, the processed signal data to a prosthetic controller. The electrical signals from the one or a plurality of VDMTs have a voltage amplitude of greater than or equal to about 50 microvolts. The prosthetic controller is configured to control a prosthetic device based on the processes signal data, preferably wirelessly, transmitted from the processing circuitry of the implant device.

In some other embodiments, this step 1520 is not required as the signals may be already received by the prosthetic controller.

In a third step 1530 the method involves analysing the received signals in term of statistical parameters in the time and frequency domain. In this step, the method may involve one or more of the following:
- amplifying the signals received by the electrodes;
- filtering the signals received by the electrodes, either before or after the amplification stage, preferably comprising at least high-pass filtering (for contact potential removal) and antialiasing (for ADC correctness);
- digitizing the amplified signals by the ADC at a sampling frequency suitable for capturing the frequency range of muscle activity (e.g. any range included in the 1 Hz- 10 kHz range, optimized by the specific muscle/implant pickup); and/or
- applying time- and frequency-domain functions to both signals to parametrise the signals received from the one or more VDMTs and the donor muscle. The system of the invention might calculate metrics such as root mean square (RMS) amplitude, mean absolute value (MAV), variance, pulse repetition frequency, and waveform length. These features help delineate differences in activation patterns between the newly reinnervated regions versus any residual electrical activity. Using methods like Fast Fourier Transform (FFT) or wavelet decomposition, the system can later identify spectral signatures unique to the signals driven by the new nerve, distinguishing them from those generated by the residual electrical activity.

Lastly, in a fourth step 1540 the method involves applying deterministic and/or deep-learning based methods to isolate the data of the electrical signals generated by the reinnervated VDMTs from at least an interference generated by the donor muscle.

Therefore, this step involves processing the composite signal received from the one or more VDMTs-encompassing both reinnervated and residual muscle activity-to isolate the data of the electrical signals generated by the reinnervated VDMTs through deterministic and/or deep-learning based methods. This allows to identify the user's intended commands - the electrical signals generated by the reinnervated VDMTs - versus extraneous or unwanted interference - generated by the donor muscle.

Because the same muscle may exhibit electrical activity from distinct sources, the fourth step may involve advanced isolation techniques. The goal is to identify and preserve the signal components specific to the newly reinnervated nerve pathway while filtering out or de-emphasizing signals from the muscle's original and remaining electrical activity.

Traditional signal processing approaches-such as adaptive filtering, principal component analysis (PCA), or independent component analysis (ICA)-may be employed to mathematically separate multiple sources of muscle activation within the same EMG signal. When the first step 1510 involves receiving synchronised electrical signals associated to: the reinnervated muscle fibres and the muscle fibres of the donor muscle, the traditional signal processing may comprise, for example, subtracting the donor muscle signal from the VDMTs signal.

More advanced embodiments utilize deep neural networks (DNNs), convolutional neural networks (CNNs), or recurrent neural networks (RNNs) to classify and isolate signals. The algorithms might be trained on labelled data where it is known that the subject has selectively activated the reinnervated nerve pathway (e.g., performing specific muscle contractions post-reinnervation). Over time, the algorithm learns characteristic features of these signals, allowing it to filter out signals that stem from residual electrical activity of the same muscle. For example, the subject may perform specific activation patterns to help the system learn and refine signal isolation, ensuring consistent performance across varied tasks. The labelled clean donor and noisy target signals, once converted to the frequency domain, can be used to train a separation neural network. This network will learn to predict the donor signal from noisy inputs, similar to the process used in speech separation to tell voices apart. The residual signal will be regarded as the clean target signal.

In some embodiments, the algorithm continuously updates its parameters-accommodating physiological changes, electrode drift, or variations in the residual muscle's activity-to maintain robust performance in isolating the reinnervated signal source.

In a particular embodiment of any embodiment of the second aspect of the invention, the method is a computer-implemented method.

In other features, the processing circuitry is configured to apply a filter as described in the previous embodiments.

Further areas of applicability will become apparent from the description provided herein. The description and specific examples in this summary are intended for purposes of illustration only and are not intended to limit the scope of the present disclosure.

### EXAMPLES

### EXAMPLE 1.

### MATERIAL AND METHODS

An anatomical study was conducted on two fresh adult cadavers, one male and one female, with a mean age at death of 78 years (ranging from 62 to 93 years). In both cases, the brachial plexus was identified, including the superior, middle, and inferior trunks, as well as the C8 and T1 roots of the inferior trunk, carefully documenting this anatomy bilaterally. Additionally, the lengths of the upper limbs and the distances between the C8 nerve root and the anterior scalene muscle (ASM) and middle scalene muscle (MSM) were measured on both sides.

### SURGICAL TECHNIQUE

### Brachial plexus dissection up to roots of C8 T1

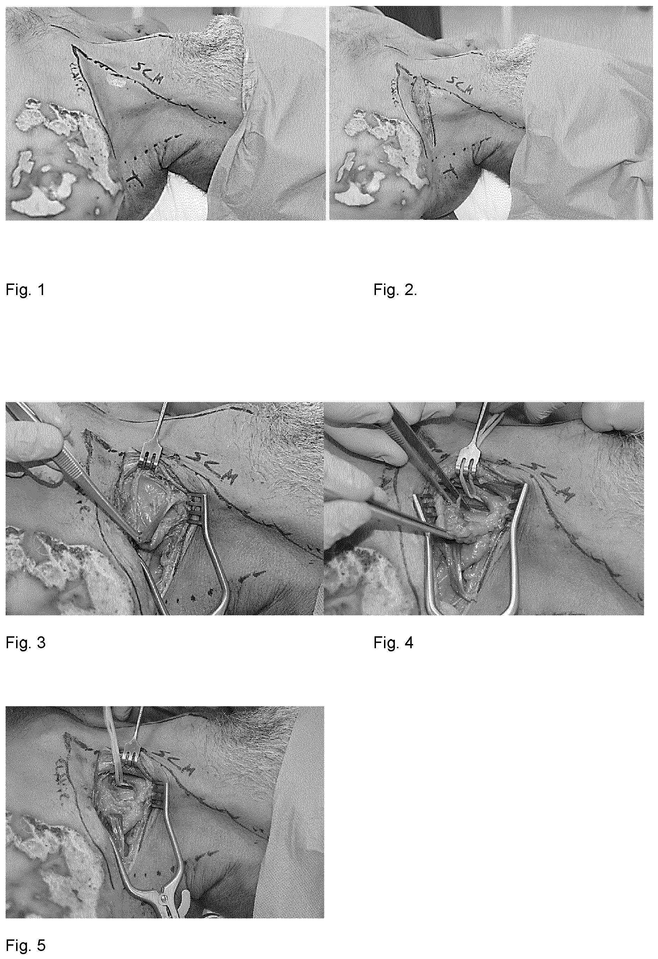

We begin by placing the patient in the supine position with the neck in maximum extension with the head oriented towards the opposite side to the one to be dissected. Then, we identified the clavicle, trapezius muscle, sternocleidomastoid muscle and mastoid process to design the skin incision (Fig 1).

In the present study we have performed the dissection through two approaches, one of them is through an transverse incision follows the superior border of the clavicle (Fig 2); and another one is through an inverted "L" shaped incision so that the vertical incision follows the posterior border of the sternocleidomastoid muscle and the transverse incision follows the superior border of the clavicle.

The first structure that we find after opening the skin and the subcutaneous cellular tissue is the platysma muscle that we will have to section in order to continue with the dissection. Next, we find the sternocleidomastoid muscle that we will have to remove superomedially. After this, the omohyoid muscle is located (Fig 3), which we must dissect and section taking care not to injure important structures such as the transverse cervical artery that runs posteriorly (Fig 4). In the present study in cadavers the omohyoid muscle and the transverse cervical artery was sectioned and then the anterior scalene muscle was identified, which is an anatomical reference structure for the location of the brachial plexus trunks. Before continuing with the dissection it is important to locate and dissect the phrenic nerve to be sure not to injure this structure during surgery (Fig 5).

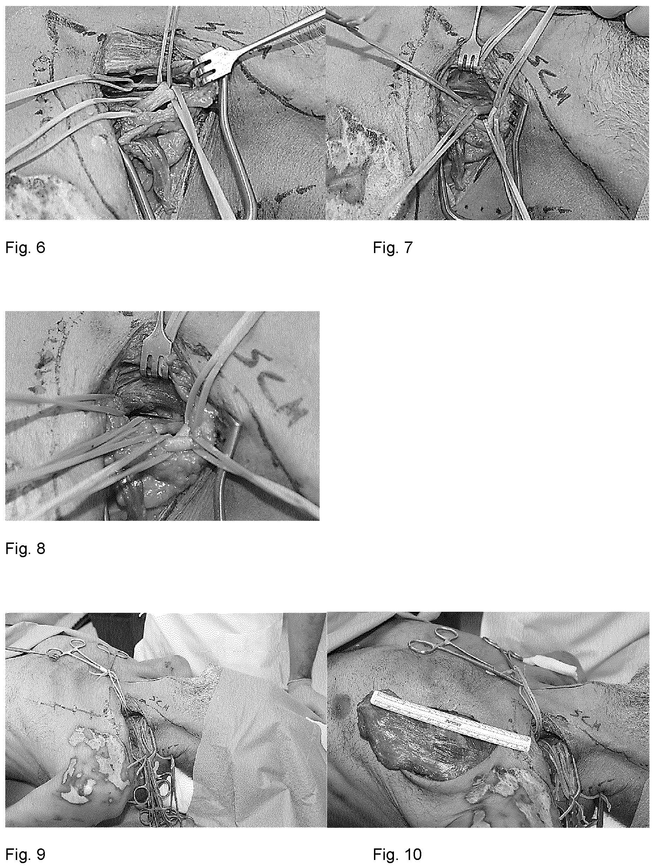

After this step, we should identify the middle scalene muscle and between this and the anterior scalene muscle we will find the superior trunk of the brachial plexus. In the course of the study we have observed that the union of the roots C5 and C6 to form the upper trunk (Fig 6) is more proximal in some patients than in others, so we must be cautious and be sure that we are dissecting the upper trunk and not one of the roots that form it; confirmation of this will be obtained by continuing the dissection in a caudal direction.

The next thing to find is the middle trunk (Fig 7) and following caudally we will identify the lower trunk. In this step we must be careful to identify the subclavian artery in order not to injure it. If we dissect proximally to the inferior trunk we will locate the C8 and T1 roots (Fig 8).

### Measurement of root attachment of C8-T1 to anterior scalene and middle scalene.

After dissection of the trunks of the brachial plexus, we measured the distance between the lower trunk and the anterior scalene and middle scalene muscles on both sides of both cadavers. The results are shown in the table 1.

### Pectoral flap dissection and measurement of distance needed for VDMT.

The pectoralis major muscle is a thick muscle located in the anterior thoracic wall whose origin is wide from several points: anterior surface of the medial half of the clavicle; lateral half of the anterior surface of the manubrium and sternum extending down to the insertion zone of the sixth or seventh costal cartilages; cartilages of the second to sixth ribs, and aponeurosis of the external oblique muscle.

It is composed of a clavicular portion whose fibers run obliquely downward and laterally; and a sternocostal portion whose fibers run upward and laterally separated by a groove to subsequently join and insert into the crest of the greater tubercle of the humerus.

The main pedicle of the sternocostal portion is the pectoral artery, one of the dominant branches of the thoracoacromial artery. It perforates the clavipectoralis fascia at a point medial to the tendon of the pectoralis minor muscle and emerges through the groove between the clavicular and sternocostal fascicles of the pectoralis major. It runs cephalocaudal to the inferior surface of the muscle, attached to it by its fascial sheath.

The dominant arterial supply to the clavicular portion is the clavicular artery, another branch of the thoracoacromial artery. It originates from the groove between the clavicular and sternocostal fascicles of the pectoralis major and runs over the inferior surface of the clavicular fascicle of the muscle.

The pectoral flap can be used in patients of all ages and in both sexes, although in women the factors of distortion of the breast shape and relatively thick subcutaneous tissue must be taken into account.

First, the pectoralis major muscle is located at the anterior thoracic wall of the patient and a longitudinal incision is designed on the skin between the middle third and the lateral third of the pectoralis major muscle (Fig 9). Then, the incision is made and dissection begins until reaching the fascia of the pectoralis major muscle. Is not necessary to dissect the entire muscle, just a muscular flap of 8 -10 cm in length (Fig 10). We start dissecting the muscle flap from the inferior border of our design in a craniocaudal direction without damaging the vascular pedicle (Fig 11).

A subcutaneous bridge is then dissected between the cranial portion of the incision over the pectoralis major and the incision on the superior border of the clavicle and it should be wider than the dissected pectoralis major flap. The muscle flap is then flipped and tunnelled to perform the insetting with resorbable suture covering the lower trunk of the dissected brachial plexus in order to suture the proximal end of the plexus to the denervated vascularized muscle (Fig 12).

Finally, a subcutaneous pocket is dissected from the lateral margin of the incision made for the dissection of the pectoralis major muscle where the device will be inserted, whose cables will be long enough to be placed under the subcutaneous tunnel following the muscle flap and inserted in the caudal portion of the pectoralis major muscle which is now located cranially over the lower trunk of the brachial plexus (Fig 13,14).

### RESULTS

The detailed anatomical measurements of the upper limbs of two cadavers, as shown in Table 1, focusing on the distance from the C8 nerve root to various anatomical landmarks and the length of the pectoral flaps.

The male cadaver (No. 1) had an upper limb length of 73 cm, with a C8-ASM distance of 1.3 cm on the right side and 1 cm on the left, as well as a C8-MSM distance of 1.8 cm and 2 cm, respectively. The pectoral flap length was 8 cm on the right side and 10 cm on the left.

In contrast, the female cadaver (No. 2) had an upper limb length of 61 cm, with a C8-ASM distance of 1.5 cm on the right side and 1.8 cm on the left, and a C8-MSM distance of 2.3 cm and 2.9 cm, respectively. The pectoral flap length in this case was 10 cm on the right side and 8.5 cm on the left.

Overall, the average length of the upper limbs in the cadavers was 67 cm, with an average C8-ASM distance of 1.4 cm, an average C8-MSM distance of 2.25 cm, and an average pectoral flap length of 9.125 cm.

These measurements provide a detailed assessment of the anatomy of the C8 roots and surrounding muscles, essential information for constructing VDMTs in patients with panplexus injuries, and offer relevant data for surgical intervention planning.

### TABLES

**Table 1. Lengths**

| **N°** | **Sex** | **Laterality** | **Upper limb length (cm)** | **C8-ASM distance (cm)** | **C8-MSM distance (cm)** | **Pectoral flap lenght (cm)** |
|---|---|---|---|---|---|---|
| 1 | Male | Right | 73 | 1,3 | 1,8 | 8 |
| 1 | Male | Left | 73 | 1 | 2 | 10 |
| 2 | Female | Right | 61 | 1,5 | 2,3 | 10 |
| 2 | Female | Left | 61 | 1,8 | 2,9 | 8,5 |

### EXAMPLE 2

### Material and methods

### Animals

Three-month- old New Zealand rabbits (Granja San Bernardo, Madrid, Spain) were used in this example (4 females and 4 males). All rabbits were caged under standard light and temperature conditions with free access to food and water throughout the study. All experimental procedures were made to minimize suffering and ensure animal welfare and were conducted in accordance with European Regulations (Directive 2010/63/EU). This study was approved by both the institutional ethics committee and the local government authority (PROEX 80.5/23; Madrid, Spain).

### Experimental design

Eight rabbits were randomly assigned to two groups, RPNI (n=4) and VDMT (n=4). In the first group, two RPNI procedures were performed in each rabbit using the peroneal and tibial nerves of the right hind limb. Donor muscle grafts were carefully isolated from the right biceps femoris and longitudinally trimmed in the direction of the muscle fibers. Each muscle graft was utilized to establish an RPNI in the recipient rabbit by neurotizing the transected proximal stump of the peroneal and tibial nerve. In the second group, a VDMT was created in each rabbit using the motor branch of the peroneal muscle. The donor muscle flaps were carefully dissected from the denervated peroneal muscle of the right hind limb. In both groups, a control group was designated in the left hind limb: a section of the motor branch of the peroneal muscle was carried out without RPNI / VDMT construction.

### Surgical technique

Rabbits were sedated with a combination of medetomidine (50 µg kg⁻¹. Sedator, Dechra; 1 mg mL⁻¹) and ketamine (10 mg kg⁻¹. Anesketin, Dechra; 100 mg mL⁻¹) intramuscularly (IM). After 15 minutes, an intravenous (IV) cannula was placed in the auricular vein and an arterial cannula was placed in the auricular artery. The rabbits were preoxygenated with 100% oxygen by mask for 3-5 minutes. General anaesthesia was induced with propofol (Propoven 2%; Fresenius Kabi) administered to effect (2-5 mg kg⁻¹) until the anaesthetic depth was deemed adequate. An IV continuous infusion with propofol (0.1-0.5 mL kg⁻¹ min⁻¹) was used for anaesthetic maintenance, while a specific laryngeal mask for rabbits (v-gel^{®} Advanced) was placed to administer oxygen during the whole procedure with a Mappleson F breathing system. Analgesia was achieved by the administration of a combination of bupivacaine (2 mg kg⁻¹. bupivacaine 0.5% Braun) and morphine (0.1 mg kg⁻¹. morphic chloride 1% Braun) by lumbosacral epidural injection (0.2 ml kg⁻¹ total volume) using an aseptic technique. Meloxicam (1 mg kg⁻¹. Meloxicam, Dechra; 20 mg mL⁻¹) was administered subcutaneously (SC) at the end of the surgery. The animals were monitored with electrocardiogram, pulse oximetry, invasive arterial pressure, capnography and body temperature by means of a multiparametric monitor (iMEC 8, Mindray). Lactated Ringer solution was administered as fluid therapy at a rate of 5 mL kg⁻¹ h⁻¹. The rabbits were placed on a heating blanket (Aposan, Cofares) to maintain body temperature between 37.5 and 38.5 °C, and eye lube (Lubrithal, Dechra; 10g) was applied to protect the eyes. Skin between the scapulae was clipped and a fentanyl patch (fentanyl matrix, Sandoz; 25 µg h⁻¹) was attached for postoperative analgesia. In addition, meloxicam (1 mg kg⁻¹) was administered SC BID for one week. Enrofloxacin (10 mg kg⁻¹ SC. Enrocill, HiFarmaX; 50 mg mL⁻¹) was administered for antibiotherapy BID for seven days.

Sedation of the rabbits for the assessment of the RPNI was achieved with an IM injection of medetomidine and ketamine (same doses used for the surgery), and IV cannula was placed in the auricular vein. Top ups of medetomidine and ketamine (one quarter of initial doses) were administered IV as deemed. Oxygen 100% was administered by mask; heart and respiratory rates, and body temperature were monitored during the procedure. Atipamezole (100 µg kg⁻¹. Vetpharma Animal Health, S.L.; 5 mg mL⁻¹) was administered SC to enhance recovery. Animals were under supervision until deemed fully recovered. Antibiotics and analgesics (enrofloxacin, fentanyl and meloxicam) were administered in a similar manner as previously stated for five further days.

The following section will detail the procedure used to create RPNI and VDMT models.

### A. RPNI

In one hind limb of each rabbit, two RPNls per animal were created using a donor muscle graft harvested from the biceps femoris muscle, measuring 10 mm in height, 5 mm in length, and 2 mm in thickness. An incision was made on the lateral aspect of the right thigh to expose the sciatic nerve within the biceps femoris. The peroneal and tibial nerves were carefully dissected and cut distally at their entrance into the lateral and medial compartments of the hind limb. Two donor muscle grafts were harvested from the right biceps femoris muscle, measuring 15 mm in height, 5 mm in length, and 2 mm in thickness, then transferred to the proximal stumps of the peroneal and tibial nerves. Each nerve stump was sutured into the muscle graft using 6-0 Prolene^{®} sutures (Ethicon, New Brunswick, NJ), and the muscle was sutured in a manner described as a "burrito" in previous rat models⁴⁶ Finally, one bipolar recording electrode and two monopolar steel needle recording electrodes (Inomed, Emmendingen, Germany) were randomly placed in the RPNI muscle of the tibial or peroneal nerve, as shown in video 2. A 4-0 Vicryl stitch was used to secure the cable to an adjacent muscle. The proximal portions of the cables from the monopolar and bipolar needles were buried in the subcutaneous abdominal wall. All surgical procedures were performed by the same team, consisting of a surgeon, neurophysiologist, and anesthesiologist.

The other hind limb of the rabbit served as the control group, where denervation of the musculature innervated by the tibial and peroneal nerves was performed. An incision was made on the lateral aspect of the left thigh to expose the sciatic nerve within the biceps femoris. The peroneal and tibial nerves were meticulously dissected and severed at their entry into the lateral and medial compartments of the leg, without any fixation of their distal ends. Subsequently, one bipolar recording electrode and two monopolar steel electrodes (Inomed, Emmendingen, Germany) were randomly placed in the previously denervated musculature of the lateral and medial compartments of the left hind limb. A 4-0 Vicryl suture was used to secure the cable to the adjacent musculature, and the proximal parts of the cables of the monopolar and bipolar electrodes were inserted into the subcutaneous abdominal wall. All surgical procedures were performed by the same team consisting of a surgeon, a neurophysiologist, and an anesthesiologist.

### B. VDMT

In one hind limb of each rabbit, a VDMT model was constructed. An incision was made on the lateral aspect of the right leg, followed by meticulous dissection to reach the plane between the biceps femoris muscle and the gastrocnemius. Through careful dissection, the motor branch of the peroneal muscle was located, confirmed by intraoperative electrical stimulation. Subsequently, the motor branch of the peroneal muscle was sectioned. Then, through an incision in the peroneal muscle, away from the previous neuromuscular junction, a muscle flap measuring 20 mm in height, 20 mm in length, and 10 mm in thickness was raised, preserving its vascularization. The distal end of the motor branch of the peroneal muscle, previously denervated, was placed within the thickness of the vascularized muscle flap and sutured to the muscle using 6-0 Prolene^{®} suture (Ethicon, New Brunswick, NJ), as described in previous publications on VDMT models 52-54. In a subsequent step, a new surgical approach was undertaken to locate the VDMT, thus enabling the placement of one bipolar recording electrode and two monopolar steel needle recording electrodes (Inomed, Emmendingen, Germany) in the gastrocnemius muscle, as demonstrated in video 3, to conduct the neurophysiological study. All surgical procedures were carried out by the same medical team, comprised of a surgeon, a neurophysiologist, and an anesthesiologist.

The other hind limb of the rabbit was designated as the control group, where denervation of the peroneal muscle was performed. An incision was made on the lateral aspect of the left leg to expose its motor branch, which was then severed and traumatized with a 6-0 Prolene^{®} suture (Ethicon, New Brunswick, NJ) at the distal end without fixation to any other tissue with the aim of avoiding spontaneous reinnervation. In a subsequent step, a new surgical approach was undertaken to locate the VDMT, thus enabling the placement of one bipolar recording electrode and two monopolar steel needle recording electrodes (Inomed, Emmendingen, Germany) in the peroneal muscle. All surgical procedures were carried out by the same medical team, comprised of a surgeon, a neurophysiologist, and an anesthesiologist.

### Electrophysiology

Nerve conduction studies were conducted at 8 weeks postoperatively to evaluate the reinnervation of the RPNIs and VDMTs, as well as their ability to transduce nerve signals.

For the studies, each rabbit was sedated and maintained at a constant body temperature of 35°C. A motor nerve conduction (MNC) study was performed following nerve stimulation, with recordings taken at the RPNI or VDMT level. After completing the 8-week measurements, the RPNI site was reopened to expose the area and confirm the correct needle placement.

Several neurophysiological variables were analysed, including the presence of electrical signals in the RPNI and VDMT, the threshold of electrical activity required to differentiate the electrical activity of the RPNI from that of distant muscles, latency, and signal amplitude. The time of appearance of the electrical signal and the temporal differences between the 4 and 8-week periods were also evaluated.

### Histology

Following endpoint testing, each RPNI was harvested and fixed in 10% formalin. Muscle tissue regeneration and reinnervation were evaluated with light microscopy. Half of the samples from each group underwent henatoxylin and eosin (H&E) staining using tissue cross-sections obtained from one-fourth and one-half the muscle lengths. The remainder were stained with Masson trichrome.

### Statistical analysis

Data are reported using the mean and standard deviation, or median and 95% confidence as indicated for continuous or ordinal variables based on variable type and normality of distribution. Categorical variables were described with counts and percentages. Statistical significance was set at P ≤ .05. All statistical analyses were performed via SPSS 25 (IBM, Armonk, NY, USA).

### Results

### A. RPNI

### Electrophysiology

In rabbits with RPNI, no muscular electrical activity was detected at 8 weeks. However, activity was recorded in the musculature distal to the RPNI, confirming the proper functioning of the EMG equipment. The results obtained are presented in Table 2.

### Histology evaluation

During macroscopic inspection, all RPNI appeared devitalized and showed signs of vascular distress. The control group exhibited bulbous swellings at the end of the transected nerve consistent with neuroma formation.

### Complications

During the study, various complications were documented, including a possible vascular incident associated with anesthesia, which resulted in paraplegia and, ultimately, the euthanasia of the rabbit due to ethical and animal welfare concerns.

### B. VDMT

### Electrophysiology

The stimulation of the VDMT nerve was performed with the corresponding electrical activity recorded. In all VDMT except in one, a response was obtained following nerve stimulation, with this response being observed at 8 weeks. The results obtained are presented in Table 2.

**Table 2**

| | **Threshold** | | **Supramaximal** | | |
|---|---|---|---|---|---|
| | **Intensity (mA)** | **Amplitude (mV)** | **Intensity (mA)** | **Amplitude (mV)** | **Latency (ms)** |
| **Rabbit #1 RPNI** | - | - | - | - | - |
| **Rabbit #2 RPNI** | - | - | - | - | - |
| **Rabbit #3 RPNI** | - | - | - | - | - |
| **Rabbit #4 RPNI** | - | - | - | - | - |
| **Rabbit #5 VDMT** | 0,2 | 3,44 | 1,6 | 4,7 | 1,2 |
| **Rabbit #6 VDMT** | - | - | - | - | - |
| **Rabbit #7 VDMT** | 1 | 0,25 | 5,8 | 0,63 | 1,6 |
| **Rabbit #8 VDMT** | 1,2 | 1,7 | 2,2 | 2,2 | 4 |

### Histology evaluation

Upon macroscopic examination, all VDMT samples exhibited healthy characteristics without signs of vascular insufficiency or muscular distress. In contrast, the negative control group displayed bulbous swellings at the transected nerve's terminus, indicative of neuroma formation.

### Complications

No complications were detected in the rabbits that underwent VDMT procedures, neither during the surgical technique nor during the postoperative follow-up.

### Discussion

The present example underscores the relevance of rabbits as an animal model in biomedical research, particularly due to their shared anatomical similarities and inflammatory responses with humans. These similarities have been well documented in the literature, supporting their use in preclinical studies aimed at translating findings to humans. Furthermore, rabbits are phylogenetically closer to primates than rodents, reinforcing their pertinence in research that requires more accurate extrapolation to humans.

The results obtained in this example revealed that tissue vascularization in the RPNI model in rabbits is limited. Given the observed challenges in tissue viability and limited neovascularization in the RPNI model, we opted to develop a vascularized RPNI model, also known as VDMT. This approach ensures adequate blood perfusion in the muscle tissue, even in the absence of innervation, improving graft viability. Preservation of vascularization is fundamental to ensuring muscle tissue survival and, consequently, the effectiveness of the VDMT model.

This VDMT model in rabbits has clear clinical implications, particularly in reconstructive surgery and the management of neuropathic pain in humans Both RPNI and VDMT aim to generate stable muscle electrical signals for application in myoelectric prostheses and to improve neuropathic pain in patients with amputations or peripheral nerve injuries. The availability of vascularized muscles near affected nerves facilitates the implementation of these procedures in patients, which could not only improve prosthetic functionality but also alleviate chronic neuropathic pain symptoms.

### Electrophysiology

Neurophysiological analysis is crucial for evaluating the functional capacity of RPNI (Resistant Nerve Muscle Wrap) and VDMT (Direct Nerve Muscle Transplant) models in generating muscle electrical signals. In this example, electromyographic measurements revealed significant differences between the RPNI and VDMT groups regarding the intensity, amplitude, and stability of the generated signals.

In the RPNI models, no electrical activity was detected. This result is largely due to the limited vascularization of the muscle tissue in these models, which impairs its viability and ability to generate consistent electrical signals. Insufficient perfusion can lead to muscle degeneration, thereby reducing the tissue's capacity to produce and maintain stable electromyographic signals.

In contrast, VDMT models showed a more stable electrical signals, with amplitude and consistency. The adequate vascularization of denervated muscles in these models allowed for better preservation of tissue structure and functionality, resulting in an enhanced ability to generate stable electrical signals. This finding is crucial for the application of these models in myoelectric prostheses, where the stability and amplitude of electromyographic (EMG) signals are key factors for precise prosthesis control.

### Histology

Histological studies of muscle tissues in VDMT models revealed proper structural organization, with well-integrated muscle tissue and evident neovascularization. However, some areas with signs of ischemia were still observed, highlighting the need to refine surgical techniques and postoperative care to further improve tissue survival. Despite these challenges, the histological results in the VDMT group were considerably superior to those observed in the non-vascularized RPNI group, where greater necrosis and muscle fibrosis were found, likely due to insufficient blood perfusion.

### Conclusions of example 2

The VDMT model demonstrates advantages over non-vascularized RPNI models, particularly in terms of improved vascularization, enhanced muscle viability, and the ability to generate stable electromyographic signals, which are essential for more effective prosthetic control. While further technical developments are required, the findings provide a strong foundation for future clinical applications.

## Claims

1. A system comprising an implant device which in turn comprises a processing circuitry and a communication circuitry, and optionally a prosthetic controller and a prosthetic device, wherein the implant device is configured to receive electrical signals from one or more Vascularized Denervated Muscle Targets (VDMTs) of a subject reinnervated with one or more nerves of the subject through one or more first electrodes, wherein the one or more electrodes are in electrical communication with the VDMTs, wherein the VDMTs pertain to a donor muscle of the subject; and
wherein the implant device is further configured to receive electrical signals from muscle fibres of the donor muscle through one or more second electrodes from the implant device,
wherein the processing circuitry is configured to process the received electrical signals to isolate the data of the electrical signals generated by the reinnervated VDMTs from at least an interference generated by the donor muscle.

2. The system according to claim 1, wherein the processing circuitry is configured to process the received electrical signals to isolate the data of the electrical signals generated by the reinnervated muscle fibres from electrical signals generated by the donor muscle by:
a. analysing the received signals in term of statistical parameters in the time and frequency domain; and
b. applying deterministic and/or deep-learning based method to isolate the data of the electrical signals generated by the reinnervated muscle fibres from at least an interference generated by the donor muscle.

3. The system according to any one of the previous claims, wherein the communication circuitry is configured to transmit the processed electrical signals to a prosthetic device.

4. The system according to claim 3, wherein the communication circuitry is further configured to transmit the processed signal data to a prosthetic controller of the prosthetic device.

5. The system according to any one of claims 3 or 4, wherein the processing circuitry is configured to generate commands to control the prosthetic device based on the processed electrical signals.

6. The system according to any one of the previous claims, wherein the one or more VDMTs is wrapped around the nerve to interact with said nerve.

7. The system according to any one of the previous claims, wherein the one or more electrodes are one or more electrodes selected from: mono-polar needle electrodes, bipolar needle electrodes, bipolar hook wire electrodes, monopolar hook wire electrodes or sub-miniature needle electrodes.

8. The system according to any one of the previous claims, wherein the donor muscle is selected from any one of the following: the peroneal muscle, the sternocleidomastoid, the omohyoid, the trapezius the rectus femoris or the gracilis.

9. A method for isolating the data of the electrical signals generated by a reinnervated VDMT of a subject, wherein the reinnervated VDMT pertain to a vascularised muscle flap of a donor muscle of the subject, and wherein the muscle fibres have been reinnervated with nerve axons from the subject, wherein the method comprises the following steps:
a. receiving synchronised electrical signals associated to: the reinnervated muscle fibres and the muscle fibres of the donor muscle;
b. analysing the received electrical signals in term of statistical parameters in the time and frequency domain; and
c. applying deterministic and/or deep-learning based methods to isolate the data of the electrical signals generated by the reinnervated muscle fibres from at least an interference generated by the donor muscle

10. Method according to claim 9, wherein the electrical signals are received by an implant device according to any one of claims 1 to 8.

11. Method according to any one of claims 9 or 10, wherein the method is a computer-implemented method.
